# EUROPEAN PATENT APPLICATION

(11) **EP 3 670 668 A1**
(43) Date of publication of application: **24.06.2020**
(21) Application number: 18382963.9
(22) Date of filing: 20.12.2018
(51) Int. Cl.: C12Q 1/6806, C12Q 1/6895

(54) **METHOD FOR THE DETECTION OF BOTRYTIS CINEREA BASED ON LOOP-MEDIATED ISOTHERMAL AMPLIFICATION, DETECTION REAGENT AND SET OF PRIMERS**

(71) Applicant: Fundación Gaiker, 48170 Zamudio Vizcaya (ES); Fundacion Neiker, 48160 Derio (Vizcaya) (ES)
(72) Inventor: Bilbao Sevillano, Ainhoa, 48170 Zamudio (Vizcaya) (ES); Olabarria de Pablo, Garbiñe, 48170 Zamudio (Vizcaya) (ES); Ortiz Barredo, Amaia, 48160 Derio (vizcaya) (ES); Díez Navajas, Ana, 48160 Derio (vizcaya) (ES)
(74) Representative: Balder IP Law, S.L.

(57) **Abstract**

The present invention relates to a method for the detection of *Botrytis cinerea* based on loop-mediated isothermal amplification (LAMP) designed to amplify specific regions of *Botrytis cinerea's* 28S rRNA gene using a set of specially designed LAMP primers, a detection reagent for specifically detecting sequences of *Botrytis cinerea's* 28S rRNA gene, and to a set of LAMP primers to amplify specific regions of *Botrytis cinerea's* 28S rRNA gene.

SEQUENCE LISTING

<110>  Fundación Gaiker

<120>  METHOD FOR THE DETECTION OF BOTRYTIS CINEREA BASED ON
       LOOP-MEDIATED ISOTHERMAL AMPLIFICATION, DETECTION REAGENT AND SET
        OF PRIMERS

<130>  P184787EP

<160>  7

<170>  PatentIn version 3.5

<210>  1
<211>  1050
<212>  DNA
<213>  Botrytis cinerea

<400>  1
aattgtgcta cacggtcctg taagtagtag agtagccttg ttgttacgat ctactgaggg      60

taagccgtcc atagcctaga tttgatttat aatctcccat ttttagcttg tccacaggct     120

tctgaaggga ttttagtgaa gtgaccgatt tcttattaaa ggaatcggtc atgttcatgt     180

gcgaaatata agctcaccgg gagcaactaa tcacattaca tatatgttgt acatatatca     240

tatgttgatt agttttcttc tctgaccaaa tcatgggccc ggtgaggtat aagaaatatt     300

tgcatctaag aagtagttgg gctatgagtg acccaagggc acgtcgagat cctgatcctc     360

gatggggacc ctaaccctaa cccggggggc cagatacgct ttaccttggt tcgactgtag     420

tccctaggaa cgccctcgga gtgtcctagg aatgcccccg gtgagccctt ggtctaaagc     480

cgtataggtg actagttaac cccatatagt ttgtgcgagt acactactac cggtgagcag     540

gctgtaattt caatgtgcag aatctgtccc cggtgagccc aggtcacctt gcaatgagtg     600

gacagcatgt ttgaaatgcg attaattgtt gctcccggtg agcccactaa ataattctga     660

gagttggcca tctcatattt catccccggt gagcccaaga tatacttacc tagaggtggt     720

aagtatggag agcaatttct cggaaattaa ttttatttag tggaatcaat tgtgggagca     780

gagcctatac aaaaggagtc gatgggtata tagaggacga gaaataaaag tatcatcgta     840

taggttgatg atatacagta tattgaggtg aggtgaggaa gatagaaacg aagagatgtg     900

aagagttgaa tatatttagg ttattaatgc tttagtatga atacgataag agtttcagta     960

agtgtatccg tagtattgag gcatcatagg ggctttgtgg gtatctgggg atgtttgggg    1020

gcctctattc ctgactagta ctatagtact                                      1050


<210>  2
<211>  19
<212>  DNA
<213>  Botrytis cienrea

<400>  2

actgtagtcc ctaggaacg                                                    19

<210>  3
<211>  18
<212>  DNA
<213>  Botrytis cinerea

<400>  3
tcaaacatgc tgtccact                                                     18

<210>  4
<211>  44
<212>  DNA
<213>  Botrytis cinerea

<400>  4
ggttaactag tcacctatac ggctttcgga gtgtcctagg aatg                        44

<210>  5
<211>  40
<212>  DNA
<213>  Botrytis cinerea

<400>  5
gcgagtacac tactaccggt gacattgcaa ggtgacctgg                             40

<210>  6
<211>  19
<212>  DNA
<213>  Botrytis cinerea

<400>  6
tagaccaagg gctcaccgg                                                    19

<210>  7
<211>  22
<212>  DNA
<213>  Botrytis cinerea

<400>  7
gcaggctgta atttcaatgt gc                                                22

## Description

### FIELD OF THE INVENTION

The present invention relates to a method based on loop-mediated isothermal amplification (LAMP) designed to specifically amplify sequences of *Botrytis cenerea's* 28S ribosomal RNA (rRNA) gene using a set of specially designed LAMP primers, to a detection reagent for specifically detecting sequences of *Botrytis cenerea's* 28S rRNA gene, and to a set of LAMP primers to amplify specific regions of *Botrytis cenerea's* 28S rRNA gene.

### BACKGROUND OF THE INVENTION

Grey mould or botrytis is caused by *Botrytis cinerea,* a single-celled microscopic fungus, and is the most important disease that attacks bunches of grapes throughout the world [1]. It is not a specific pathogen of the vine since it can attack many other wild and cultivated plants, such as fruits and vegetables, producing irreversible damage in the harvest and significantly reducing the quality of the fruit. The grapes will most commonly be damaged by the time of harvest when the pathogen attacks the bunch very quickly, but the infection can occur at any time during the growing season. The cycle of the disease has three different stages depending on the season of the year [2]. During wet springs it can directly infect the stems and leaves or the flowers causing latent infections.

The fungus hibernates mainly in the form of sclerotia, formed in the autumn in the branches, as well as in the form of mycelium in the bark and buds. It also grows in multiple species of plants and decomposing plant materials forming important sources of inoculum that later spread in the air through the spores. In spring, sclerotia and mycelium produce conidia, forms of resistance, which are the sources for a new cycle of infection [3].

The penetration of the conidia into the plant occurs through the stomata, or through micro-wounds or natural openings that in the case of the grape can be caused by mechanical damage such as hail, insect feeding or small cracks during the maturation. Once inside the tissues the fungus produces the necrosis or decomposition of the same, after which a mycelium becomes visible on the outside, which produces the conidia that are initially white but after a few days acquires a grayish color characteristic of the disease. The fungus attacks all of the green organs of the vine, but mainly the bunches. In wet springs, infected buds and shoots turn brown, necrotic and dry.

There are four moments that are considered critical for the control of the disease: end of flowering, closure of the bunch, beginning of ripening and 21 days before the harvest. According to the recommendations in the Guide for the Integral Management of Pests, 2014 [3], the treatments with phytosanitary products should be carried out in a preventive way, considering the meteorological conditions, mainly the humidity, the vigor of the crop and the varietal sensitivity. It is precisely in this context, and taking into account that the spores of the fungus are dispersed through the air to the tissue of the grape, where a direct measurement of the presence of spores in different environmental locations of the vineyard allows the producer to make decisions about a sustainable use of chemical compounds by considering not only the most appropriate time but also the plot where the treatments are in fact necessary.

In general, filamentous fungi are the main pathogens of plants that cause a loss of around 20% of crops worldwide [4]. The economic losses caused by botrytis are very high and difficult to quantify, both because of the wide range of hosts it colonizes (more than 200 varieties), and because it is capable of infecting the crop almost at any stage of its life cycle. *Botrytis cinerea* is one of the main pathogenic fungi of grapes for wine production, being responsible for the greatest losses in viticulture.

The economic impact derived from gray mould is disastrous in the cultivation of wine, as well as in other crops such as strawberries and/or bulbous plants. It is estimated that it produces a loss of 15-40% of the harvests, depending on weather conditions, exceeding 15,000 million euros per year (25% of the global potential of turnover of wine production).

The fight against botrytis requires the use of fungicides, modifications of cultivation techniques and the development of more resistant serovars. The treatments with phytosanitary products envolve near of 540 million euros annually, which represent 10% of the world-wide market of the sale of fungicides. There are two issues associated with the use of these products in the different phases of the cultivation of the grapes for transformation:
1. On the one hand, *Botrytis cinerea* has developed resistance to multiple fungicides of common use, and although the magnitude of this problem is not yet well documented, there are studies that show that genetic mutations are produced which result in greater resistance and consequently to a loss of efficacy of fungicides. Overuse of phytosanitary products along with the introduction of new classes of fungicides have led to the emergence of new multi-resistant strains in several countries [5];
2. Moreover, some of the fungicides, such as iprodione, procymidone, captan or chlorothalonil, are intensively used, sometimes with inadequate overdosing in order to ensure their efficiency. This malpractice can lead to the persistence of undesirable chemical residues in the grapes, which in turn can be transferred to the wine [6] and compromise the safety of the product [7]. In addition to the risks to the consumer's health, which may be due to inappropriate use of pesticides in wine, they may also modify the development of winemaking and the sensory quality of the product [8].

Several studies show that residues of chemical products, especially fungicides, present in wine grapes can damage the fermentation process and affect its quality. It has been demonstrated that the yeast *Saccharomyces cerevisiae* is affected by the presence of phytosanitary residues, in such a way that the alcoholic fermentation can be delayed, prevented or stopped. If this occurs, the wort is left unprotected and consequently other microorganisms, such as acetic bacteria can colonize the broth causing a lactic and/or acetic pitting. Also, the presence of residual fungicides can alter by themselves the balance of certain compounds responsible for the sensory quality of wine, in general, and aroma, in particular.

According to the Directive 2009/128/EC the use of fungicides in vineyards must be necessarily justified based on objective parameters taking into account local circumstances, i.e. taking into account the circumstances of the risk of infection in each plot, which is commonly referred to as integrated pest management (IPM). In this context, it is imperative to develop alternative strategies that allow the risk of infection to be evaluated in an early, localized and reliable way in order to increase the efficacy of phytosanitary products and apply them only to the risk plots. Thus, it is expected to decrease the use of these treatments quantitatively and qualitatively.

The strategies of integrated control of the disease botrytis [10] in the vineyard include several agronomical factors (fertilization, varietal sensitivity and timing (and type) of pruning), but in all wine-growing regions the use of phytosanitary products is inevitable under favourable meteorological conditions for the development of the fungus. The incidence and severity of the disease will, however, be conditioned not only by meteorological conditions but also by the amount of inoculum (propagules, spores in this case) that is present in the air and comes into contact with the sensitive parts of the vine.

The monitoring of the risk of disease based on meteorological data is not reliable because climate change makes a precise prediction difficult. Therefore, this predictive monitoring must be complemented with the capture of spores and real-time monitoring of the presence of botrytis in vineyards and grape must after harvest, so that botrytis prevention in grape can be addressed in its entirety, from the cultivation phase to the production of the wine.

### SUMMARY OF THE INVENTION

The present inventors have developed a fast, easy-to-use and reliable molecular test for the detection of the fungus *Botrytis cinerea* that is capable of detecting as many *Botrytis cinerea* strains as possible with a high level of sensitivity, precision and specificity. The test can be implemented on-site allowing for an early detection of the fungus in vine and grapes, thereby significantly reducing the incidence of grey mould in vine and grapes. The developed test is key for the prevention of the disease, and provides for a significant reduction in the use of phytosanitary products and increase in the quality of the wine due to the absence of compounds derived from the fungus. Furthermore, the method can be integrated as a tool for making decisions in the control of the disease in the field and as a quality control system for musts in the cellar.

The developed method comprises a rapid procedure for the extraction of the genetic material followed by the isothermal amplification of a molecular region of *Botrytis cinerea* corresponding to the gene coding for 28S rRNA. To this end, a set of oligonucleotides specifically designed to amplify the 28S rRNA region of *Botrytis cinerea* has been used differentially from other possible microorganisms present in the sample. The isothermal reaction known as Loop-Mediated Isothermal Amplification (LAMP) allows rapid detection of the fungus and a colorimetric reading of the result with naked eye that allows to determine the presence or absence of the fungus, without the need for specific laboratory equipment.

Accordingly, a first aspect of the invention relates to a method for the detection of *Botrytis cinerea* based on loop-mediated isothermal amplification characterized in that it comprises the steps of:
(a) providing a sample;
(b) extraction of genetic material from the sample with an extraction reagent comprising at least cellular lysis reagents and a magnetizable support, and elution of the genetic material from the magnetizable support;
(c) loop-mediated isothermal amplification with a detection reagent comprising at least an amplification solution, said amplification solution comprising a set of LAMP primers to amplify specific regions of *Botrytis cinerea's* 28S rRNA gene; and
(d) result reading,
   wherein the set of LAMP primers to amplify specific regions of *Botrytis cinerea's* 28S rRNA gene in step (c) comprises at least:
   a forward primer F3 comprising a nucleotide sequence as set forth in SEQ ID No. 1;
   a backward primer B3 comprising a nucleotide sequence as set forth in SEQ ID No. 2;
   a forward inner primer FIP comprising a nucleotide sequence as set forth in SEQ ID No. 3; and
   a backward inner primer BIP comprising a nucleotide sequence as set forth in SEQ ID No. 4.

A second aspect of the present invention relates to detection reagent for specifically detecting sequences of *Botrytis cinerea's* 28S rRNA gene, characterized in that it comprises at least an amplification solution comprising a set of LAMP primers to amplify specific regions of *Botrytis cinerea's* 28S rRNA, wherein the set of LAMP primers to amplify specific regions of *Botrytis cinerea's* 28S rRNA gene comprises at least:
a forward primer F3 comprising a nucleotide sequence as set forth in SEQ ID No. 1;
a backward primer B3 comprising a nucleotide sequence as set forth in SEQ ID No. 2;
a forward inner primer FIP comprising a nucleotide sequence as set forth in SEQ ID No. 3;
a backward inner primer BIP comprising a nucleotide sequence as set forth in SEQ ID No. 4.

A third aspect of the present invention relates to a set of LAMP primers to amplify specific regions of *Botrytis cinerea's* 28S rRNA gene, characterized in that it comprises at least:
a forward primer F3 comprising a nucleotide sequence as set forth in SEQ ID No. 1;
a backward primer B3 comprising a nucleotide sequence as set forth in SEQ ID No. 2;
a forward inner primer FIP comprising a nucleotide sequence as set forth in SEQ ID No. 3;
a backward inner primer BIP comprising a nucleotide sequence as set forth in SEQ ID No. 4.

### DRAWINGS

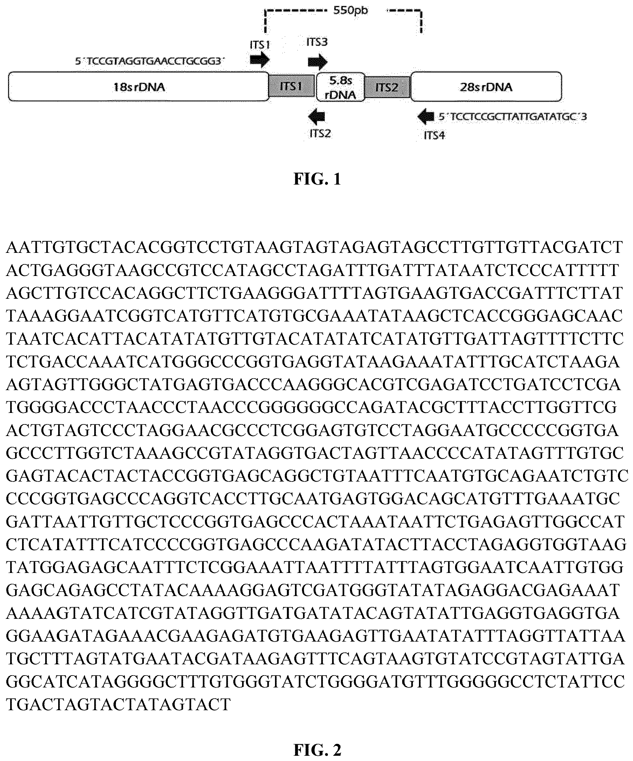

FIG. 1: Representation of the location of the 18S rRNA ribosomal gene of the fungus, the regions of the transcribed internal spacers (ITS) and the primer binding regions.
FIG. 2: Nucleotide sequence of *Botrytis cinerea's* 28S rRNA gene, intergenic spacer and 18S rRNA gene, strain SAS56 GenBank: AM233400.1.

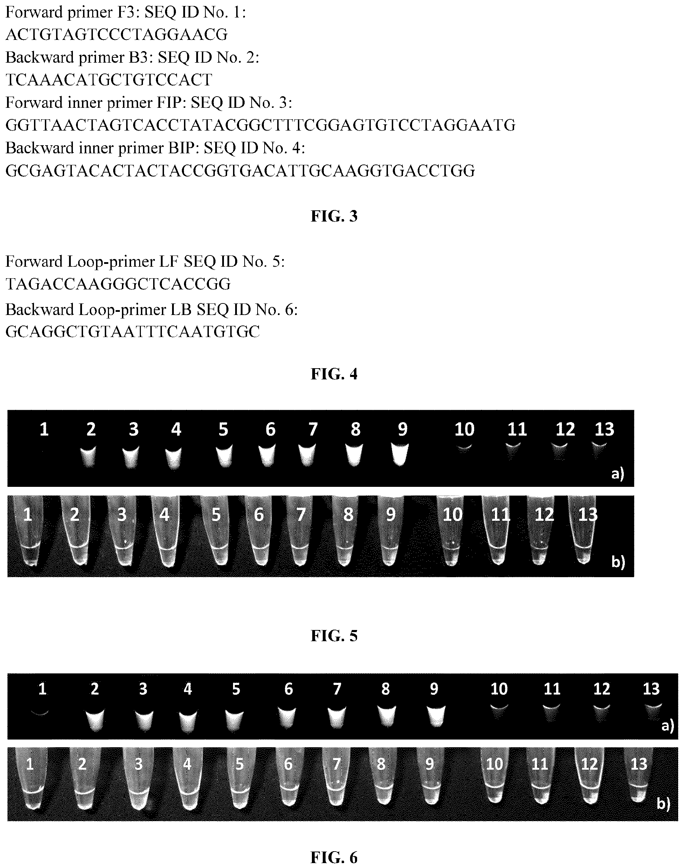

FIG. 3: Nucleotide sequence of the set of LAMP primers to amplify specific regions of *Botrytis cinerea's* 28S rRNA gene.
FIG. 4: Nucleotide sequence of Loop-primers to amplify specific regions of *Botrytis cinerea's* 28S rRNA gene.
FIG. 5: Photograph of isothermal amplification results obtained with the Bst large enzyme. Negative control of the reaction (tube 1), different strains of *B. cinerea* (tubes 2-9) and exclusive fungi; *Aspergillus fumigatus* (tube 10), *Penicillium chrysogenum* (tube 11), *Cladospoides cladosporioides* (tube 12) and *Alternaria alternata* (tube 13) detected by fluorescence (a) and by colour change in the presence of calcein (b).
FIG. 6: Photograph of the isothermal amplification results obtained with the Bst 2.0 enzyme. Negative control of the reaction (tube 1), different strains of *B. cinerea* (tubes 2-9) and exclusive fungi; *Aspergillus fumigatus* (tube 10), *Penicillium chrysogenum* (tube 11), *Cladospoides cladosporioides* (tube 12) and *Alternaria alternata* (tube 13) detected by fluorescence (a) and by colour change in the presence of calcein (b).

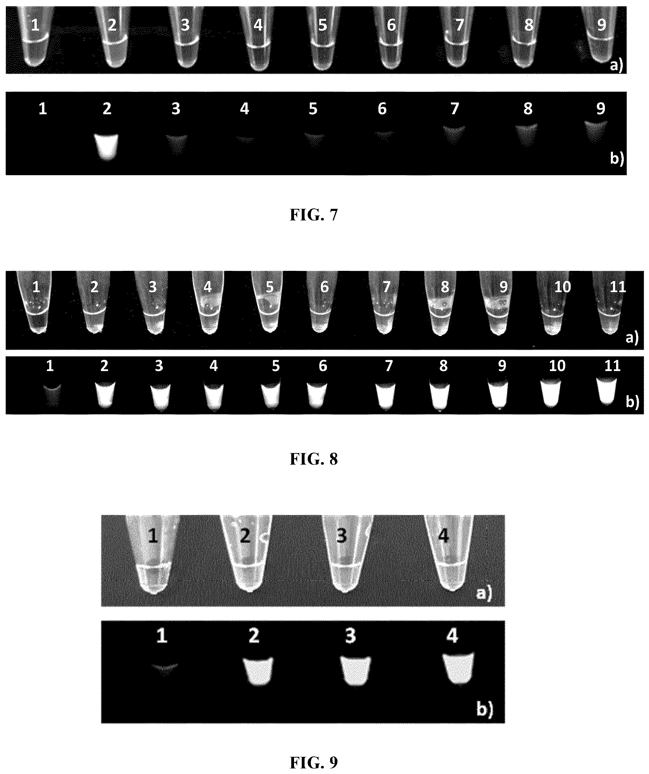

FIG. 7: Photograph of the results of isothermal amplification obtained with the Bst 2.0 enzyme. The samples are *B. cinerea* (tube 1) as positive control and; exclusive fungi and bacteria: *Uncicula necator* (tube 2), *Fusarium oxyporum* (tube 3), *Aspergillus terreus* (tube 4), *Alternaria alternata* (tube 5), *Penicillium chrysogenum* (tube 6) *Cladospoides cladosporioides* (tube 7) and *Pseudomonas aeruginosa* (tube 8) and the negative control of the reaction (tube 9). All of them detected by fluorescence (a) and by colour change in the presence of calcein (b).
FIG. 8: Photograph of isothermal amplification results obtained for the negative reaction control (tube 1), and samples with different calibrated quantities of DNA of *Botrytis cinerea* containing 17 ng (tubes 2-3), 1.7 ng (tubes 4-5), 170 pg (tubes 6-7), 17 pg (tubes 8-9) and 1.7 pg (tubes 10-11); detected by colorimetric (a) and fluorescence emission (b).
FIG. 9: Photograph of isothermal amplification results obtained after NA extraction of calibrated *Botrytis cinerea* spores. The negative control of the reaction (tube 1), 70 spores and 7 spores (tubes 2 and 3); respectively, and a positive control (tube 4) of a *Botrytis cinerea* pure DNA extract.

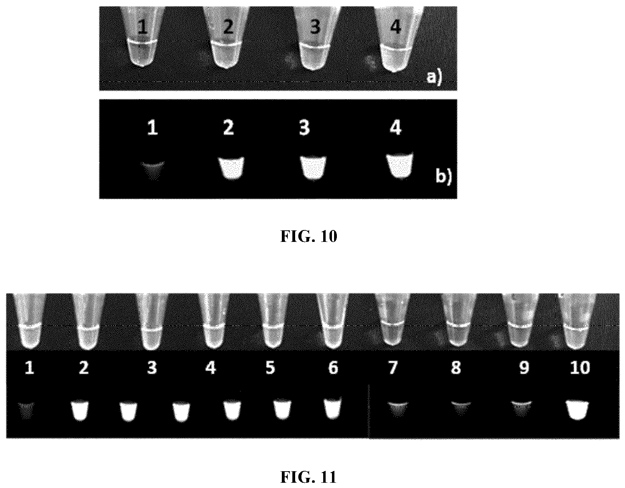

FIG. 10: Photograph of isothermal amplification results obtained after sample treatment and NA extraction from grape-must doped with calibrated *Botrytis cinerea* spores. Negative reaction control (tube 1), samples containing 140 *B. cinerea* spores (tubes 2 and 3); and the positive control of a pure DNA extract (tube 4); analysed by colorimetric (a) and fluorescence emission (b).
FIG. 11: Photograph of the results of isothermal amplification obtained for the negative control of the reaction (tube 1), the positive control (tube 10) and extracts of grapes contaminated with *Botrytis cinerea* (tubes 2-6) and uncontaminated grapes (tubes 7-9), analysed by colorimetric (a) and fluorescence emission (b).

### DETAILED DESCRIPTION OF THE INVENTION

### Method for the detection of Botrytis cinerea based on loop-mediated isothermal amplification

### (a) Providing a sample

A sample can be any plant species or any product derived from a plant species that can be affected by *Botrytis cinerea.*

According to one embodiment of the present invention, the sample is grape or must.

### (b) Extraction of genetic material from the sample with an extraction reagent comprising at least cellular lysis reagents and a magnetizable support, and elution of the genetic material from the magnetizable support

According to the present invention, the extraction of the genetic material comprises the following steps:
1. Cell lysis by addition of a lysis reagent, wherein the lysis reagent comprises at least an enzyme capable of cellular lysis and a magnetizable support. The lysis reagent reacts with the cell wall of the spore producing its lysis or breakage and releasing the nucleic acids. The nucleic acids (DNA and RNA) are captured by the magnetizable support by a magnetic field generated by a magnet, preferably a neodymium.
2. Elution of nucleic acids (DNA and RNA) by heating in order to recover the genetic material released from the magnetizable particles in the elution reagent.

The two above-mentioned extraction stages are carried out simultaneously in a single test tube. The experimental optimization of the genetic material extraction step in order to obtain an efficient separation of the nucleic acids from the cellular debris and from the magnetizable support was performed by studying different variables, such as the amount of magnetic micro-particles used, the concentration of the enzyme that favours the lysis of the cell wall, temperature and time of action of the magnetic field for a good capture and elution of the captured genetic material.

In principle, any enzyme capable of cellular lysis may be used. Suitable enzymes according to the invention comprise, but are not limited to: chitinase, glucanase, protease, cellulase, amylase or proteinase K. In a preferred embodiment of the invention a complex of endoglucanase and protease, preferably lyticase is used.

In a preferred embodiment of the present invention the at least one enzyme capable of cellular lysis is comprised in an amount between 50 to 500 units. More preferably, the at least one enzyme capable of cellular lysis is comprised in an amount between 100 to 300 units, more preferably 200 units.

After the cell walls of the spores are ruptured the nucleic acids are released and captured by the magnetizable support present in the extraction reagent. The nucleic acids are retained on the surface of said magnetizable support and, thus, separated from the rest cell debris and liquid waste

Accordingly, in principle any magnetizable support capable of trapping nucleic acids may be used.

In one embodiment of the present invention the magnetizable support comprises magnetic micro-beads.

The magnetic micro-beads are comprised in the extraction solution in an amount between 100 µl and 300 µl, preferably 150 µl and 250 µl, and more preferably 200 µl.

In one embodiment, the spores are incubated with the magnetizable support preferably at room temperature during 0 to 15 minutes, preferably 0 to 10 minutes, more preferable 0 to 5 minutes, most preferably 5 minutes, in the presence of the at least one enzyme capable of cellular lysis.

In another embodiment, the magnetizable support is concentrated under the action of the magnetic field, preferably at room temperature, during 0 to 6 minutes, preferably 0 to 4 minutes, more preferably 0 to 2 minutes, most preferably 2 minutes.

The elution of the genetic material is done with DNAase and RNAase free-grade water under heating, preferably to a temperature of 50 °C to 75°C, more preferably 55°C to 70°C, most preferably 65°C, during 0 to 20 minutes, preferably 0 to 15 minutes, more preferably 15 minutes.

In a preferred embodiment of the present invention, the genetic material is extracted by the addition of 200 µl of magnetic micro-beads and incubation at room temperature during 5 minutes in the presence of 200 units lyticase; concentration of the magnetic micro-beads under the action of a magnetic field for 2 minutes at room temperature; and elution of the genetic material with DNAase and RNAase free-grade water by heating to 65°C during 15 minutes.

The extraction reagent optionally further comprises at least one washing buffer and at least one chelating agent.

Suitable washing buffers according to the invention comprise, but are not limited to Tris-HCl buffers with a pH from 5.5 to 8.5, or 10 mM Tris-HCl (pH 7.5), 1 mM EDTA, 2M NaCl, (optional) 0.1%Tween 20 or 0.15 M NaCl, 20 mM Tris-HCl (pH 7.5), 1 mM EDTA.

### (c) Loop-mediated isothermal amplification with a detection reagent comprising at least an amplification solution, said amplification solution comprising at least one set of LAMP primers to amplify specific regions of Botrytis cinerea's 28S rRNA gene

The method for the detection of *Botrytis cinerea* according to the present invention is based on loop-mediated isothermal amplification (LAMP). Consequently, according to the invention, the isothermal amplification of specific regions of *Botrytis cinerea's* 28S rRNA gene occurs at a constant temperature. This allows carrying out the molecular assay at a unique temperature without the requirement of temperature cycles.

Another achievement of the method according to the present invention is the reagents cocktail that allows to carry out the retrotranscription and amplification steps simultaneously and in the same place, as well as reaching a sensitivity of only 7 spores of *Botrytis cinerea* and a detection limit of 1,7 pg of *Botrytis cinerea.*

For the design of the isothermal amplification, a bibliographic search was made of the genetic markers used for the detection of the species *Botrytis cinerea* by other molecular techniques. The detection strategy chosen is the isothermal amplification of the ribosomal gene (rRNA), which has been previously referenced for the detection of *Botrytis* by another molecular technique known as polymerase chain reaction or PCR (*Polymerase Chain Reaction*). Its interest as a marker is because it is a highly conserved genomic region that possesses a good specificity for the segment in which the 45S nuclear ribosomal cistron is located. This cistron is made up of ribosomal genes 18S, 5.8S and 26/28S, two internal spacers that are transcribed (ITS-1 and ITS2), and an intergenic spacer (IGS) that includes at both ends two transcribed external spacers (ETS-1 and ETS-2). This is the basic 45S unit that together with the other nuclear 5S ribosomal gene, produce the mature RNA of the subunits of the cytoplasmic ribosomes in most eukaryotes (FIG. 1).

According to the present invention, the loop-mediated isothermal amplification is performed with an amplification solution comprising at least one set of LAMP primers to specifically designed to amplify the 28S rRNA region of *Botrytis cinerea* differentially from that of other possible microorganisms present in the sample.

Suitable amplification solutions according to the invention are those capable of amplifying a single copy or a few copies of a piece of DNA across several orders of magnitude. In this case cDNA, because it was synthetized from an RNA template.

The amplification solution according to the invention further comprises at least one DNA polymerase, at least one at least one DNA stabilizer, and at least one cofactor.

DNA polymerases suitable according to the invention are those demonstrating a robust performance even in high concentrations of amplification inhibitors and featuring significantly increased reverse transcriptase activity.

Among these inhibitors, commonly present in vineyards and also in must, are phytosanitary products that are commonly used in crops to eradicate diseases and pests.

In a preferred embodiment the DNA polymerase is a *BST* DNA polymerase, preferably *Bst* 2.0 and *Bst* large.

In one embodiment, the at least an DNA polymerase is comprised in a range between 5 and 20 units. Preferably, the DNA polymerase is comprised in a range between 10 and 15 units, more preferably 12 units.

According to an embodiment of the invention, the at least one DNA stabilizer is elected from the group consisting of glycerol, polyethylene glycol, formamide, betaine, 7-deaza-dGTP, dimethyl Sulfoxide and dITP. In a preferred embodiment, the DNA stabilizer is betaine.

In one embodiment, the at least one DNA stabilizer is comprised at a concentration between 0,4 and 2,0 M. Preferably, the at least one DNA stabilizer is comprised at a concentration between 1,0 and 1,5 M, more preferably 1,0 M.

According to a preferred embodiment of the invention, the at least one cofactor is selected from the group consisting of: MgSO₄, 2-(trimethylazaniumyl) acetate, or mixtures thereof.

In one embodiment, the at least one cofactor is comprised at a concentration between 1 mM and 10 mM. Preferably, the at least one cofactor is comprised at a concentration between 2 mM and 6 mM, more preferably 4 mM.

In an embodiment, the amplification solution comprises an amplification mixture comprising at least an isothermal polymerase, at least one DNA stabilizer, at least one cofactor, a deoxyribonucleotide triphosphate (dNTP) mixture and the set of LAMP primers designed to amplify specific regions of *Botrytis cinerea* 28S rRNA gene.

In one embodiment, the isothermal polymerase is comprised in a range of 10 to 15 units, the at least one DNA stabilizer is comprised in a range of 0,4 and 2 M. the at least one cofactor is comprised in a range of 1 mM and 10 mM, the dNTP mixture at a concentration between 1,0 to 2,3 mM each one, and the set of LAMP primers to amplify specific regions of *Botrytis cinerea* 28S rRNA gene in an average concentration between 150 nM and 6.500 nM

### Set of LAMP primers to amplify specific regions of Botrytis cinerea 28S rRNA gene

The specific LAMP primers have been designed on the basis of *Botrytis cinerea* 28S rRANA gene, intergenic spacer and 18S rRNA gene, strain SAS56, GenBank: AM233400 (FIG. 2).

An "*in silico*" homology study was performed to delimit, within the selected region, the area to be used for the design of the primers. This study was carried out with different sequenced strains of the species *Botrytis cinerea,* inclusive strains, and with exclusive microorganisms, which are species of fungi and bacteria that can be found in the vineyards and must, but which are not the target of detection of the test described in the present patent. All of them, inclusive and exclusive microorganisms, are listed in Table 1.

**Table 1: List of inclusive and exclusive fungi used for the study of homologies.**

| **Inclusive Fungi** | **Exclusive Fungi** | **Exclusive Bacteria** |
|---|---|---|
| *Botrytis cinerea* (different strains) | *Aspergillus fumigatus* | *Pseudomonas aeruginosa* |
| | *Aspergillus terreus* | |
| | *Penicillium chrysogenum* | |
| | *Penicillium purpurugenum* | |
| | *Alternaria alternata* | |
| | *Cladosporium* | |
| | *cladosporioides* | |
| | *Fusarium oxysporum* | |
| | *Rhizopus* spp | |

After this analysis of homologies, a fragment of the subunit 28S rRNA, intergenic spacer and 18S rRNA was selected to proceed with the design of primers with the help of a specific computer program for the loop mediated isothermal amplification (LAMP). Eight sets of different primers were designed, of which two were experimentally tested in order to determine the best candidate in terms of performance and sensitivity.

The set of primer to amplify specific regions of *Botrytis cinerea* 28S rRNA gene according to the invention comprises at least four primers:
a Forward primer F3 comprising a nucleotide sequence as set forth in SEQ ID No. 1;
a Backward primer B3 comprising a nucleotide sequence as set forth in SEQ ID No. 2;
a Forward inner primer FIP comprising a nucleotide sequence as set forth in SEQ ID No. 3; and
a Backward inner primer BIP comprising a nucleotide sequence as set forth in SEQ ID No. 4.

FIG. 3 shows the nucleotide sequences of the LAMP-primers according to the present invention.

Thus, according to the present invention the amplification solution comprises a set of LAMP primers to amplify specific regions of *Botrytis cinerea* 28S rRNA gene comprising at least:
a Forward primer F3 comprising a nucleotide sequence as set forth in SEQ ID No. 1;
a Backward primer B3 comprising a nucleotide sequence as set forth in SEQ ID No. 2;
a Forward inner primer FIP comprising a nucleotide sequence as set forth in SEQ ID No. 3; and
a Backward inner primer BIP comprising a nucleotide sequence as set forth in SEQ ID No. 4.

According to one embodiment of the present invention, the amplification solution comprises preferably 1.500 nM and 250 nM of the two pairs of isothermal amplification primers, respectively.

In order to accelerate the molecular reactions, another pair of "Loop-primers" can be added:
Forward Loop-primer LF comprising a nucleotide sequence as set forth in SEQ ID No. 5;
Backward Loop-primer LB comprising a nucleotide sequence as set forth in SEQ ID No. 6.

FIG. 4 shows the nucleotide sequence of Loop-primers to amplify specific regions of Botrytis cinerea's 28S rRNA gene.

According to one embodiment of the present invention, the amplification solution comprises preferably 750 nM of the "Loop-primers".

Any nucleic acid sequences listed herein or in the accompanying sequence listing are shown using standard letter abbreviations for nucleotide bases.

Along the isothermal amplification of the nucleic acids, the reaction yields high quantities of pyrophosphate as by-product of the enzymatic reaction. This pyrophosphate reacts with the magnesium ions present in the reaction mixture. These complexes can be dyed with specific fluorophores, in the Frequency Amplitude Modulated (FAM) spectrum, which can develop a colour change in the reaction mixture, that can be detected in the visible spectrum at the same time that the reaction takes place. A direct reading of the reaction with naked eye is thus possible.

Accordingly, in a preferred embodiment of the invention, the amplification solution further comprises at least one reading reagent comprising a Frequency Amplitude Modulated (FAM) spectra fluorophore.

Suitable FAM spectra fluorophores according to the invention comprise, but are not limited to: calcein, hydroxyl naphthol blue (HNB), 6-carboxyfluorescein, Alexa fluor 488, Fluorescein FITC. In a preferred embodiment of the invention the FAM spectra fluorophore is calcein.

In one embodiment, the at least one reading reagent is comprised in a concentration between 10 to 100 mM reaction volume. Preferably, the reading reagent is comprised in a concentration between 20 to 80 mM, more preferably 40 mM.

In an embodiment of the present invention, the detection reagent comprises an amplification mixture comprising at least an isothermal polymerase, at least one DNA stabilizer, at least one cofactor, a deoxyribonucleotide triphosphate (dNTP) mixture and the set of LAMP primers designed to amplify specific regions of *Botrytis cinerea* 28S rRNA gene and at least one reading reagent comprising a Frequency Amplitude Modulated (FAM) spectra fluorophore.

In an embodiment, the detection reagent comprises an isothermal polymerase in a range of 10 to 15 units, at least one DNA stabilizer in a range of 0,4 and 2 M., at least one cofactor in a range of 1 mM and 10 mM, a dNTP mixture at a concentration between 1.0 to 2.3 mM, a set of LAMP primers to amplify specific regions of *Botrytis cinerea* 28S rRNA gene in an average concentration between 150 nM and 6.500 nM, and at least one reading reagent comprising a Frequency Amplitude Modulated (FAM) spectra fluorophore in a concentration of 10 and 100 mM .

### (d) Result reading

As mentioned above, the presence specific fluorophores in the detection reagent allows for the direct reading of the reaction with naked eye: a) in the visible by means of colorimetric change by precipitation of the pyrophosphate generated during the amplification reaction in the presence of a substrate, such as calcein, producing a colour change from orange to intense yellow during the course of the amplification reaction, b) in the fluorescent when excitation of the solution occurs at a wavelength of 496 nm, an increased fluorescence emission is generated in the fluorescent green, which increases in intensity as the DNA polymerizes and c) in the visible by means of colorimetric change by the production of protons and the subsequent pH drop which generates a colour change of the solution from pink to yellow.

According to the present invention, preferred methods for results reading are in the visible by using calcein for the detection with naked eye of the colour change, and in the fluorescent, the visualization at the end of the differential fluorescence emission reaction. These reading methods allow for a simpler and immediate interpretation of the results and consequently for a rapid on-site detection of *Botrytis cinerea.*

### EXAMPLES

### 1. Specificity results

As shown in Table 2, a number of experimental tests were carried out in order to determine the specificity of the assay for different microorganisms: different inclusive strains of *Botrytis cinerea,* and other genera of exclusive fungi, such as *Alternaria, Cladosporium, Penicillium, Aspergillus* and *Fusarium,* usually present in the cultivation of grapes for winemaking, and of bacteria present in the environment, such as *Pseudomonas aeruginosa.*

**Table 2: List of inclusive and exclusive tested fungi tested and identification of the test tubes.**

| **Tube identification** | **Positive Results** | **Negative Results** |
|---|---|---|
| 1 | | Negative Control |
| 2 | *Botrytis cinerea*-9 | |
| 3 | *Botrytis cinerea*-12 | |
| 4 | *Botrytis cinerea*-14 | |
| 5 | *Botrytis cinerea*-16 | |
| 6 | *Botrytis cinerea*-17 | |
| 7 | *Botrytis cinerea*-31 | |
| 8 | *Botrytis cinerea*-32 | |
| 9 | *Botrytis cinerea*-35 | |
| 10 | | *Aspergillus fumigatus* CECT 20228 |
| 11 | | *Penicillium chrysogenum* CECT 2306 |
| 12 | | *Cladospoides cladosporioides* CECT 20805 |
| 13 | | *Alternaria alternata* DSM 62010 |

Figures 5 and 6 show the specificity results obtained for the set of primers according to the present invention with the *Bst* large polymerase enzymes (Fig. 5) and *Bst* 2.0 (Fig. 6). As can be seen all the strains of *Botrytis cinerea* give a positive result, when the result reading is performed by fluorescence: fluorescence emission of tubes 2 to 9, and absence of fluorescence emission, both for negative control of the reaction and for the exclusive microorganisms; tubes 1 and 10 to 13, respectively, of Fig. 5a and 6a. In the same way, in the analysis of the colorimetric detection a colour difference between the positive tubes was detected, intense yellow colour, and the negative tubes, orange colour (Fig. 5b and 6b).

In Figure 6, the specificity results obtained for the set of primers according to the invention with the Bst 2.0 polymerase enzyme are shown. As can be seen, all the strains of *Botrytis cinerea* give a positive result, both in fluorescence detection, fluorescence emission from tubes 2 to 9, and absence of fluorescence emission both for negative control of the reaction and for the exclusive microorganisms; tubes 1 and 10 to 13, respectively, of Fig 6a. For colorimetric detection, a colour difference was also observed between the positive tubes, intense yellow colour, and the negative tubes, orange, Fig. 5b.

In the specificity study further exclusive fungi were included (see Table 3), in order to ensure the specificity of the detection method of the invention.

**Table 3: List of inclusive and exclusive microorganisms with the identification of the test tubes.**

| **Tube identification** | **Positive Results** | **Negative Results** |
|---|---|---|
| 1 | *Botrytis cinerea* | |
| 2 | | *Uncinula necator* |
| 3 | | *Fusarium oxysporum* CECT 2159 |
| 4 | | *Aspergillus terreus* CECT 2808 |
| 5 | | *Alternaria alternata* DSM 62010 |
| 6 | | *Cladospoides cladosporioides* CECT 20805 |
| 7 | | *Cladosporium cladosporioides* CECT 20805 |
| 8 | | *Pseudomonas aeruginosa* CECT 116 |
| NTC | | Negative Control |

Figure 7 shows that all the exclusive microorganisms tested (tubes from 2 to 8) by fluorescence (a) and colorimetry (b) do not give an amplification signal, thus no colour change nor a differential emission of fluorescence with respect to the negative control (tube 1) where the DNA extract of a *Botrytis cinenea* collection strain has been amplified, is observed.

### 2. Determination of the limit of detection

The limit of detection of the isothermal amplification technique has been established from *Botrytis cinerea* DNA calibrated at different concentrations. The correlation between Cq (time at which the real-time fluorescence reading curve begins to grow exponentially) and the initial amount of DNA was also studied. Table 4 shows the results of Cq against decreasing amounts of *Botrytis cinerea* DNA. Likewise, the gene equivalents or theoretical genomic copies corresponding to each calibrated quantity of DNA are detailed.

**Table 4: Cq data obtained for different quantities of DNA of Botrytis cinerea measuring the emission of fluorescence in real time, reading every minute, and the correlation of tubes of each determination.**

| DNA (pg) | Cq1 | Cq2 | E.G. | Tube |
|---|---|---|---|---|
| Negative | N/A | | | 1 |
| 17.000 | 32,53 | 32,08 | 1,9 x 10⁻⁵ | 2, 3 |
| 1.700 | 34,75 | 34,30 | 1,9 x 10⁻⁴ | 4, 5 |
| 170 | 37,70 | 37,93 | 1,9 x 10⁻³ | 6, 7 |
| 17 | 41,18 | 41,29 | 1,9 x 10⁻² | 8, 9 |
| 1,7 | 44,20 | 64,31 | 1,9 x 10⁻¹ | 10, 11 |

According to these data, the limit of quantification where a linear correlation between DNA quantity and fluorescence emission data (Cq) is maintained is 17 pg of DNA, which is equivalent to 190 copies of *Botrytis.* Therefore, there are four logarithmic linear correlation orders between these 17 ng and 17 pg with an R² of 0.99. The determined limit of detection is 1,7 pg of DNA, which is equivalent to 19 copies. Figure 8 shows the colorimetric and fluorescence emission result for the different quantities tested.

In order to establish the sensitivity of the complete technique; extraction stage based on magnetic micro-particles and isothermal amplification stage, calibrated suspensions of *Botrytis cinerea* spores equivalent to 70 and 7 spores per test were used. The results are shown in Figure 9 and were obtained with an amplification time of only 30 minutes.

It is demonstrated that the detection method according to the invention is able to detect only 7 spores of *Botrytis cinerea* with a simple procedure, without the need to use laboratory equipment and with a detection system that allows an immediate interpretation of the results.

### 3. Experimental study with grape and must samples

It has been shown that the developed method detects the presence of *Botrytis* in contaminated samples of grape must and in the grape itself. To this end, very simple pre-treatments were established for the sample in order to proceed with the detection method according to the present invention.

### 3.1 Detection from a must sample

The must from crushed grapes is doped with 140 calibrated spores of *Botrytis cinerea.* The solution is left to decant at room temperature in 2 ml tubes in order to eliminate the remains coming from the pulp and wall of the must grape that could interfere with the detection test.

Subsequently, the soluble phase of the must of two independent samples M1 and M2 was analysed by the isothermal detection test according to the invention. Figure 9 shows the results of the two samples and compares both, the colour shift and the differential fluorescence emission with respect to a positive control, calibrated *B. cinerea* DNA (tube 1) and the negative reference control, absence of genetic material (tube 4) The result is positive for the two samples since it coincides both in colour and fluorescence emission with the positive control.

The same process was repeated with grape must samples naturally contaminated with *Botrytis cinerea* and the obtained results were the same.

### 3.2 Detection from grapes

Grape seeds contaminated with *Botrytis* and uncontaminated grapes were used as a negative control of the detection test according to the invention.

The grape grains were pre-treated with 1 ml of saline in which the spores of the fungus were collected. 0.5 ml of wash was used to complete the detection test including isothermal extraction and amplification. The results of all the samples tested are shown in Figure 11. As can be seen, all of the contaminated tested grape grains gave a positive result while the uncontaminated ones gave negative results.

It is, therefore, confirmed that *Botrytis cinerea* can be detected in both, grape and must with very simple pre-treatments and by means of the detection test according to the present invention.

### References:

[1] Damon Smith et al. Washington State University Extension; April 11, 2012. http://articles.extension.org/pages/32279/botritis-pudricin-del-racimo-o-moho-gris-en-uvas-botrytis-bunch-rot-or-gray-mold-of-grapes.
[2] Michelle Moyer, Washington State University Extension; Feb, 2015.
[3] Guía de Gestión Integradas de Plagas-Uva de transformación. Ministerio de Agricultura, Alimentación y MedioAmbiente; Madrid, 2014.
[4] http://www.genoscope.cns.fr/spip/Botrytis-cinerea-estimated-losses.html
[5] Matthias Hahn "The rising threat of fungicide resistance in plant pathogenic fungi: Botrytis as a case study" J Chem Biol (2014) 7:133-141.
[6] González-Rodriguez, R. M. Cancho-Grande, B. Torrado-Agrasar, A. Simal-Gándara, J. Mazaira-Pérez, J. 2009. "Evolution of tebuconazole residues through the winemaking process of Mencía grapes". Food Chemistry, 117, 529-537.
[7] Rose, G. Lane, S. Jordan, R. 2009. "The fate of fungicide and insecticide residues in Australian wine grape by-products following field application". Food Chemistry, 117, 634-640.
[8] Patil, S. H. Banerjee, K. Dasgupta, S. Oulkar, D. P. Patil, S. B. Jadhav, M. R. Savant, R.H. Adsule, P. G. Deshmukh, M. B. 2009. Multiresidue analysis of 83 pesticides and 12 dioxin-like polychlorinated biphenyls in wine by gas chromat.
[9] Almeida, G. 2010. "Cultura da macieira no Rio Grande do Sul: análise situacional e descrição varietal". Embrapa Uva e Vinho, Rio Grande do Sul, Brasil. Dirección URL:
[10] Katherine J. Evans. "Integrated management of botrytis bunch rot fact sheet grape and wine research and development corporation", January 2010.
[11] Simona M. Sanzani et al. "Early detection of Botrytis cinerea latent infections as a tool to improve postharvest quality of table grapes". Postharvest Biology and Technology 68 (2012) 64-71.

## Claims

1. A method for the detection of *Botrytis cinerea* based on loop-mediated isothermal amplification **characterized in that** it comprises the steps of:
(a) providing a sample;
(b) extraction of genetic material from the sample with an extraction reagent comprising at least cellular lysis reagents and a magnetizable support, and elution of the genetic material from the magnetizable support;
(c) loop-mediated isothermal amplification with a detection reagent comprising at least an amplification solution, said amplification solution comprising a set of LAMP primers to amplify specific regions of *Botrytis cinerea's* 28S rRNA gene; and
(d) result reading,
wherein the set of LAMP primers to amplify specific regions of *Botrytis cinerea's* 28S rRNA gene in step (c) comprises at least:
a forward primer F3 comprising a nucleotide sequence as set forth in SEQ ID No. 1;
a backward primer B3 comprising a nucleotide sequence as set forth in SEQ ID No. 2;
a forward inner primer FIP comprising a nucleotide sequence as set forth in SEQ ID No. 3; and
a backward inner primer BIP comprising a nucleotide sequence as set forth in SEQ ID No. 4.

2. The detection method according to claim 1, wherein the set of LAMP primers to amplify specific regions of *Botrytis cinerea's* 28S rRNA gene in step (c) further comprises a forward loop-primer comprising a nucleotide sequence as set forth in SEQ ID No. 5 and a backward loop-primer comprising a nucleotide sequence as set forth in SEQ ID No 6.

3. The method according to claims 1 or 2, wherein the amplification solution in step (c) comprises at least one reading reagent comprising a Frequency Amplitude Modulated (FAM) spectra fluorophore.

4. A detection reagent for specifically detecting sequences of *Botrytis cinerea's* 28S rRNA gene, **characterized in that** it comprises at least an amplification solution comprising a set of LAMP primers to amplify specific regions of *Botrytis cinerea's* 28S rRNA, wherein the set of LAMP primers to amplify specific regions of *Botrytis cinerea's* 28S rRNA gene comprises at least:
a forward primer F3 comprising a nucleotide sequence as set forth in SEQ ID No. 1;
a backward primer B3 comprising a nucleotide sequence as set forth in SEQ ID No. 2;
a forward inner primer FIP comprising a nucleotide sequence as set forth in SEQ ID No. 3; and
a backward inner primer BIP comprising a nucleotide sequence as set forth in SEQ ID No. 4.

5. The detection reagent according to claim 4, wherein the set of LAMP primers to amplify specific regions of *Botrytis cinerea's* 28S rRNA gene further comprises a forward loop-primer comprising a nucleotide sequence as set forth in SEQ ID No. 5 and a backward loop-primer comprising a nucleotide sequence as set forth in SEQ ID No 6.

6. The detection reagent according to claim 4 or 5, wherein said detection reagent comprises at least one reading reagent comprising a Frequency Amplitude Modulated (FAM) spectra fluorophore.

7. A set of LAMP primers to amplify specific regions of *Botrytis cinerea's* 28S rRNA gene, **characterized in that** it comprises at least:
a forward primer F3 comprising a nucleotide sequence as set forth in SEQ ID No. 1;
a backward primer B3 comprising a nucleotide sequence as set forth in SEQ ID No. 2;
a forward inner primer FIP comprising a nucleotide sequence as set forth in SEQ ID No. 3; and
a backward inner primer BIP comprising a nucleotide sequence as set forth in SEQ ID No. 4.

8. The set of LAMP primers according to claim 7, wherein the set of LAMP primers to amplify specific regions of *Botrytis cinerea's* 28S rRNA gene further comprises a forward loop-primer comprising a nucleotide sequence as set forth in SEQ ID No. 5 and a backward loop-primer comprising a nucleotide sequence as set forth in SEQ ID No 6.
